# EUROPÄISCHE PATENTSCHRIFT

(11) **EP 1 618 854 B1**
(45) Veröffentlichungstag und Bekanntmachung des Hinweises auf die Patenterteilung: **04.09.2019**
(21) Anmeldenummer: 05106658.7
(22) Anmeldetag: 20.07.2005
(51) Int. Cl.: A61C 13/00, A61C 8/00

(54) **Verfahren und Vorrichtung zur Bestimmung der Lage und Orientierung der Achse eines direkt im Patientenmund befindlichen dentalen Implantats**
Method and device for determining the position and the orientation of the axis of an implant present in the mouth of a patient
Procédé et dispositif de détermination de la position et de l'orientation de l'axe d'un implant situé dans la bouche d'un patient

(30) Priorität: 20.07.2004 DE 102004035091
(43) Veröffentlichungstag der Anmeldung: 25.01.2006
(73) Patentinhaber: Sirona Dental Systems GmbH, 64625 Bensheim (DE)
(72) Erfinder: Saliger, Günter, 64625, Bensheim (DE); Orth, Ulrich, 64686, Lautertal (DE); Teckentrup, Steffen, 64646, Heppenheim (DE); Pieper, Reinhard, 64625, Bensheim (DE)
(74) Vertreter: Özer, Alpdeniz

(56) Entgegenhaltungen:
- EP-A- 0 599 578
- WO-A-03/024352
- WO-A-2004/060197
- US-A1- 2002 039 717
- US-B1- 6 287 119

## Beschreibung

### Technisches Gebiet

Die Erfindung betrifft das Gebiet der prothetischen Versorgung mit zahnärztlichen Implantaten und insbesondere die praktische Ausgestaltung von Vermessung, Konstruktion und Herstellung von Suprastrukturen mit Hilfe von CAD/CAM Geräten.

Die prothetische Versorgung von zahnärztlichen Implantaten ist heute eine Aufgabe, die der Zahnarzt zusammen mit seinem Dentallabor löst. Die obere Kante eines enossalen zahnärztlichen Implantats liegt typisch in etwa auf der Höhe des Kieferknochens oder schließt mit ihm bündig ab. Die Höhenunterschiede zwischen dem Implantatkopf und der Okklusalfläche der zu dem Implantat benachbarten Zähne kann dabei insbesondere im inzisalen und prämolaren Bereich so groß werden, dass die beiden Strukturen mit optischen Vermessungssystemen auf Grund begrenzter Schärfentiefe nicht mehr gleichzeitig mit ausreichender Genauigkeit vermessen werden können. Das Problem wird noch dadurch verstärkt, dass der zahnlose Kieferknochen nach kurzer Zeit degeneriert und die besagten Abstände sich dadurch weiter vergrößern.

Die Oberkante des Implantats liegt darüber hinaus in einem Bereich, der regelmäßig von Speichel und anderen Körperflüssigkeiten umspült wird und in dem Drüsen liegen, die verschiedene Sekrete erzeugen. Damit ist es schwierig, den Kopf des Implantats so trocken zu halten, dass er direkt im Mund des Patienten vermessen werden kann.

Der Implantatkopf ist regelmäßig so konzipiert, dass eine mechanisch hochwertige Verbindung mit geringem Spiel, hoher Passung und gutem Kraftschluss zu der Suprastruktur bzw. einem Abutment entsteht. Das ergibt geometrische Ausgestaltungen am Implantatkopf, die z. B. mit herkömmlichen intraoralen Messverfahren nur schwer in der erforderlichen Genauigkeit vermessen werden könnten. Dies gilt insbesondere dann, wenn der Implantatkopf gepudert wird. Das Problem wird durch einen ungleichmäßigen Puderauftrag noch verstärkt.

Das Implantat ist von Gingiva bzw. Knochensubstanz umgeben. Beide Oberflächen können nur unter Schwierigkeiten so bepudert werden, dass sie nach mit herkömmlichen intraoralen Messverfahren vermessen werden können.

Die Erfindung betrifft daher insbesondere ein Verfahren zur Bestimmung der Lage und Orientierung der Achse eines direkt im Patientenmund befindlichen dentalen Implantats gemäß Anspruch 1 sowie eine Vorrichtung zur Durchführung dieses Verfahrens gemäß Anspruch 6.

### Stand der Technik

Zur Versorgung eines im Patientenmund befindlichen Implantats ist bisher die Herstellung eines Modells nötig. Dabei werden unter anderem die Lage und die Orientierung des Implantats in einem aufwendigen Verfahren über eine Abdrucknahme auf das Arbeitsmodell des Zahntechnikers übertragen. Alle weiteren Aufbauarbeiten finden dann an diesem Modell statt.

Die Herstellung von Abutments und Kronen für Implantate ist daher heute eine Leistung, die ausschließlich von einem zahntechnischen Labor durchgeführt wird.

Der Zahnarzt hingegen setzt die Implantate. Nach einer Einheilphase nimmt er einen Abdruck von der entstandenen Situationen. Dabei überträgt er die Position der Implantate durch die Einbringung von Repliken in den Abdruck. Mit Hilfe eines analogen Verfahrens überträgt der Zahntechniker dann die Lage dieser Implantate auf sein Meistermodell. Danach nutzt der Zahntechniker das Angebot an Abutments, das der jeweilige Hersteller des verwendeten Implantats anbietet. Die Abutments werden eventuell noch den Erfordernissen der Situation manuell angepasst, bevor der Zahntechniker in konventioneller Weise eine Krone anfertigt. Abutment und Krone werden in weiteren Arbeitsschritten dem Patienten eingesetzt.

Aus der EP-A2-1 062 916 ist ein Verfahren zur Herstellung eines Zahnersatzes sowie ein Verfahren zur Herstellung eines Zahnersatzteiles bekannt, welches von der Erstellung eines Negativabdruckes des Kiefers ausgeht und anhand eines daraus hergestellten Arbeitsmodells das Setzen und Vermessung von Implantaten zur Konstruktion von Gerüsten ermöglicht. Die dort gegebenen Definitionen und Beschreibungen des grundsätzlichen Ablaufes zur Vorbereitung einer Vermessung bzw. zur anschließenden Konstruktion und Herstellung werden vollumfänglich in die vorliegende Anmeldung mit einbezogen.

Das dort beschriebene Verfahren beruht darauf, dass in einen konventionellen Abdruck ein sogenanntes ManipulierImplantat eingebracht wird und damit auf dem Modell eine Situation hergestellt wird, wie sie bereits im Mund des Patienten nach der Inkorporation des Implantats vorliegt. Diese klinische Situation wird dann mit Hilfe eines Scanners vermessen mit dem Ziel, Abutment und Suprastruktur herzustellen. Zur Lagebestimmung des Implantats wird ein Hilfselement verwendet.

Mit diesem Verfahren werden die Arbeiten, die der Zahntechniker auch heute schon nach dem Stand der Technik ausführen muss, computerisiert, d.h. die zu erstellenden Zwischenstufen Modellierung von Abutment, Gerüst und Verblendung werden digitalisiert, um sie hinterher auszuschleifen.

Aus der EP 1 062 916 A2 ist weiterhin ein Arbeitsmodell mit eingebauten Manipulierimplantaten bekannt, an welchem ein Positionierungselement mit einem für die Vermessung geeigneten Hilfselement vorgesehen ist. Aus den Messdaten werden Implantatdaten ermittelt, die zur Konstruktion einer Suprastruktur in Form eines Abutments verwendet werden.

Nachteilig hierbei ist, dass erst ein Modell erstellt werden muss, um die Lage und Orientierung der Achse eines dentalen Implantats in der klinischen Situation des Kiefers zu erfassen und erst anschließend einen Implantataufbau durchzuführen. Die vorgeschlagenen Verfahren erfordern eine Trennung zwischen der Tätigkeit des Zahnarztes am Patienten selbst und in die Tätigkeit eines Zahntechnikers an einem Modell. Eine Anwendung der Verfahren am Patienten selbst ist nicht möglich.

Verfahren zur Bestimmung der Lage und Orientierung der Achse eines dentalen Implantats sind auch aus den EP 599 578 A2, US 6,287,119 B1 und WO 03/024352 A1 bekannt.

Aufgabe der Erfindung ist es, eine Vorrichtung und ein Verfahren anzugeben, mit der es möglich wird die Konstruktion und Herstellung der benötigten Suprastrukturen direkt und sofern gewünscht unmittelbar am Patienten ohne Einschaltung eines Dentallabors bzw. Zahntechnikers mit einem CAD/CAM-Gerät auszuführen oder die notwendigen Arbeitsschritte des Zahntechnikers rationeller als bisher zu gestalten.

### Darstellung der Erfindung

In den Ansprüchen wird ein Verfahren und eine Vorrichtung vorgestellt, die direkt in der klinischen Situation im Patientenmund anwendbar ist. Diese klinische Situation kann mit einer Messkamera optisch vermessen werden, sodass Messdaten vorliegen. Aus diesen Messdaten können dann alle notwendigen Daten über Lage und Orientierung der Achse des dentalen Implantats ermittelt werden. Danach ist die Erstellung einer Implantatsuprastruktur mit CAD/CAM-Verfahren möglich.

Bei dem Verfahren zur Bestimmung der Lage und Orientierung der Achse eines sich direkt im Patientenmund befindlichen dentalen Implantats werden Messdaten, welche durch optisches Vermessen der klinischen Situation des Implantats im Patientenmund gewonnen wurden, durch geeignete Verfahren, beispielsweise der Bildverarbeitung, analysiert, um die Lage und die Orientierung einer Messgeometrie zu bestimmen. Dazu ist an dem Implantat eine Messgeometrie angebracht, wobei die Messgeometrie derart ausgebildet ist, dass sie einen Rückschluss auf die Lage und Orientierung des Implantats zulässt. Anhand der Lagebestimmung der Messgeometrie wird die relative Lage und Orientierung zwischen der Achse des dentalen Implantats und der optischen Achse der Vermessung bestimmt und anhand der Messgeometrie wird die Lage und die Orientierung des Implantats selbst bestimmt.

Der Vorteil liegt darin, dass die klinische Situation direkt im Patientenmund erfasst wird, sodass sich die Herstellung eines Modells erübrigt.

Vorteilhafterweise ist die Messgeometrie an einem Vermessungsteil mit einer Anschlussgeometrie für das Implantat angebracht und wird aus der Messgeometrie die relative Lage und Orientierung der Anschlussgeometrie des Implantats bestimmt. Dadurch ist der Einsatz von handelsüblichen Implantaten mit den üblichen Anschlussgeometrien für die herzustellende Suprastruktur möglich. Darüber hinaus kann die Messgeometrie unabhängig von der Anschlussgeometrie gestaltet werden.

Erfindungsgemäß wird die klinische Situation des Implantats und der Nachbarzähne in den Messdaten analysiert und eine Achse relativ zur Achse der Vermessung bestimmt. Diese Achse kann die Einschubachse des oberen Teils der Implantatsuprastruktur, beispielsweise einer Krone sein, aber auch eine an der Okklusionsfläche orientierte Achse, die dann vorzugsweise senkrecht dazu steht. Es ist auch möglich, diese Achse manuell vorzugeben oder bei Betrachtung des Messdatensatzes durch den Benutzer durch diesen auswählen zu lassen.

Mit der so bestimmten bzw. festgelegten Achse ist es möglich, die Einschubrichtung einer Krone relativ zu der Implantatachse zu definieren. Damit wird es möglich, die wesentlichen Bestimmungsstücke des Abutments als Teil der Implantatsuprastruktur festzulegen, nämlich Art, Lage und Orientierung des Implantatanschlusses, Höhe und Form des Abutmentaufbaus und zervikale Grenze des Abutments.

Das hier vorgeschlagene Verfahren ist zur Bestimmung der Lage und Orientierung des Ausgleichswinkels einer Implantatsuprastruktur zur Verbindung mit einem direkt im Patientenmund befindlichen dentalen Implantat vorteilhafterweise so weitergebildet, dass der Ausgleichswinkel bestimmt wird, der sich aus den gemessenen Winkeln zwischen der Achse des dentalen Implantats und der Achse der Vermessung und zwischen der Achse der Vermessung und der Achse ergibt, die durch die Okklusalebene oder die Morphologie des zu ersetzenden Zahns selbst festgelegt ist. Die Implantatsuprastruktur selbst kann dabei einteilig oder mehrteilig sein.

Anstelle der Bezeichnung "Ausgleichswinkel" wird auch die Bezeichnung "Raumwinkel" verwendet.

Gemäß einer Weiterbildung wird die Implantatsuprastruktur mit dem bestimmten Ausgleichswinkel hergestellt, insbesondere mit CAD/CAM-Verfahren.

Vorteilhafterweise weist die Messgeometrie mindestens einen Teilbereich auf, der so ausgebildet ist, dass sich aus der Analyse dieses Teilbereichs die Art des vorhandenen Implantats ergibt. Für verschiedene Implantate werden in diesem Fall Vermessungsteile mit verschiedenen Messgeometrien eingesetzt.

Vorteilhafterweise weist die Anschlussgeometrie des Implantats mindestens einen Teilbereich auf, der so ausgebildet ist, dass sich aus der Analyse dieses Teilbereichs die Art des vorhandenen Implantats ergibt.

Ein weiterer Gegenstand der Erfindung ist eine Vorrichtung, zur Durchführung des vorstehenden Verfahrens, bei welcher in einer Auswerteeinheit Messdaten durch geeignete Verfahren analysiert werden und die Lage und Orientierung einer Messgeometrie bestimmt wird. Die Messdaten wurden dabei durch Vermessen der klinischen Situation eines Implantats und der Nachbarzähne im Patientenmund gewonnen. Dabei wurde an dem Implantat eine Messgeometrie angebracht, die einen Rückschluss auf die Lage und Orientierung einer Anschlussgeometrie des Implantats zulässt. In der Auswerteeinheit wird anhand der Lagebestimmung der Messgeometrie die relative Lage und Orientierung zwischen der Achse des dentalen Implantats und der Achse der Vermessung bestimmt. Darüber hinaus wird in der Auswerteeinheit ausgehend von der Messgeometrie die Anschlussgeometrie und damit die Lage und die Orientierung des Implantats selbst bestimmt.

Dabei kann sowohl ein Implantat mit integrierter Messgeometrie verwendet werden als auch ein zusätzliches Vermessungsteil vorgesehen sein.

Erfindungsgemäß werden in der Auswerteeinheit die klinische Situation oder Nachbarzähne in den Messdaten analysiert und eine Achse relativ zur Achse der Vermessung bestimmt. Weiterhin wird in der Auswerteeinheit ein Ausgleichswinkel bestimmt, der sich aus den gemessenen Winkeln zwischen der Achse des dentalen Implantats und der Achse der Vermessung und zwischen der Achse der Vermessung und der Achse der Okklusalebene ergibt. Schließlich ist eine Bearbeitungseinheit vorgesehen, mit welcher die Implantatsuprastruktur, oder bei mehrteiligen Strukturen zumindest das Anschlussteil an das Implantat mit dem bestimmten Ausgleichswinkel herstellbar ist.

Vorteilhafterweise weist die Messgeometrie oder die Anschlussgeometrie des Implantats mindestens einen die Art des vorhandenen Implantats ergebenden Teilbereich auf. Dies kann durch geometrische Formgebung, Beschriftung oder farbliche Gestaltung erfolgen.

Ein weiterer Gegenstand ist ein Aufsatzteil und ein Verfahren zur Anwendung des Aufsatzteils.

Ein Aufsatzteil auf ein in einem Kiefer eingebrachtes Implantat oder ein in einem Arbeitsmodell vorgesehenes Manipulierimplantat weist eine Positionierungsvorrichtung und Befestigungsmittel zum Implantat hin sowie weiterhin einen eine Codierung aufweisenden Teilbereich auf. Zwischen der Positionierungsvorrichtung und dem Teilbereich ist ein Distanzstück vorgesehen, dessen Länge in dem Teilbereich codiert ist. Dadurch ist es möglich, durch Vermessen des Aufsatzstückes die Lage des Implantatkopfes festzustellen.

Vorteilhafterweise ist die Länge des Distanzstückes so bemessen, dass der Teilbereich auf das Niveau der Okklusalfläche der Nachbarzähne gebracht ist.

Gemäß einer Weiterbildung weist die Oberfläche der Teilbereiche einen hohen Rückstreukoeffizienten mit einer Lambert'schen Streuverteilung sowohl im sichtbaren wie im nahen Infrarotbereich des Spektrums auf. Dabei kann die Oberfläche beschichtet sein.

Vorteilhafterweise ist ein gegenüber dem Distanzstück bewegliches Ausgleichsteil vorgesehen, dass das Aufsatzteil umfangsseitig umschließt und Auflageflächen zur Auflage auf dem die Zahnrestauration umgebenden Zahnfleisch aufweist. Dadurch kann die zu vermessende Stelle frei von Blut und Speichel gehalten werden. Darüber hinaus kann das Ausgleichsteil als Ersatzmodell für den Verlauf des Zahnfleisches angesehen werden.

Vorteilhafterweise ist das Ausgleichsteil auswechselbar und kann somit angepasst an die tatsächliche Restaurationsstelle in Größe und Form ausgewählt werden.

Dabei kann das Ausgleichsteil in seiner Orientierung einstellbar sein und eine Kennung aufweisen, die eine Bestimmung der Orientierung ermöglicht.

Das beschriebene Verfahren zur Vermessung von einem in einem Kiefer eingebrachten Implantat oder von einem in einem Arbeitsmodell vorgesehenen Manipulierimplantat, wobei an dem Implantat ein Aufsatzteil positioniert und befestigt ist und wobei über einen eine Codierung aufweisenden Teilbereich des Aufsatzteils auf die Lage des Implantates im Kieferbereich geschlossen werden kann umfass weiterhin die Verwendung eines Aufsatzteils, welches mit einem zwischen der Positionierungsvorrichtung und dem Teilbereich vorhandenen Distanzstück an dem Implantat oder Manipulierimplantat befestigt wird, wobei die Länge des Distanzstückes in dem Teilbereich codiert ist. Nach der Vermessung und der Erstellung eines 3D-Datensatzes ist die Länge des Distanzstückes durch Auswertung des codierten Teilbereichs bestimmbar.

Weitere Ausgestaltungen des Verfahrens sind in den dazugehörigen Unteransprüchen wiedergegeben.

### Kurzbeschreibung der Zeichnung

Das erfindungsgemäße Verfahren wird anhand der Zeichnung erläutert. Es zeigen:
- Fig. 1: eine klinische Situation eines im Patientenmund befindlichen Implantats mit Nachbarzähnen, die
- Fig. 2: einen Querschnitt durch ein Vermessungsteil, die
- Fig. 3a: eine Draufsicht auf eine Anschlussgeometrie des Implantats, die
- Fig. 3b: ein weiteres Implantat mit einer Anschlussgeometrie und einer Messgeometrie, die
- Fig. 4: eine Schemazeichnung einer erfindungsgemäßen Vorrichtung, die
- Fig. 5: eine Implantatsuprastruktur in Form eines Abutments, die
- Fig. 6: eine typische Präparationsstelle für eine Zahnrestauration mit einem Aufsatzteil im Querschnitt und die
- Fig. 7: ein Aufsatzteil in einer Seitenansicht. Ausführungsbeispiel

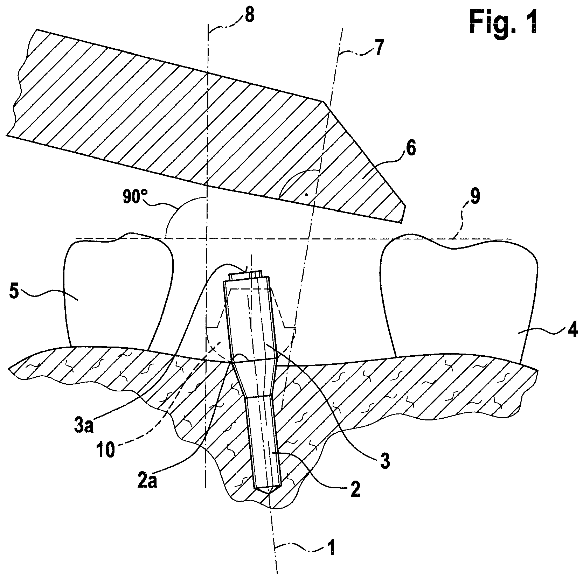

Anhand der Fig. 1 wird das Verfahren zur Erfassung der Lage und Orientierung der Achse 1 eines dentalen Implantats 2 erläutert. Zunächst wird ein Vermessungsteil 3 mit einer Messgeometrie 3a auf das direkt im Patientenmund befindliche Implantat 2 gesteckt. Anschließend wird die klinische Situation, gebildet durch das Implantat 2 und vorhandene Nachbarzähne 4, 5 optisch vermessen, z.B. mit einer Messkamera 6. Drei verschiedene Achsen, die Richtungen festlegen, sind zu diesem Zeitpunkt relevant: die Achse 1 des dentalen Implantats, eine optische Achse 7 der Messkamera 6 und die unter Berücksichtigung der Nachbarzähne 4, 5 bestimmte Achse 8 des Zahns, die beispielsweise als Einschubachse verwendet wird. In dem Fall, dass die Nachbarzähne 4, 5 Backenzähne sind, liegt die Achse 8 senkrecht zu einer gedachten Okklusionsfläche 9.

Zur Bestimmung der Zahnachse 8 können zusätzlich dem System z.B. aus einer Zahnbibliothek bekannte Beschaffenheiten des zu ersetzenden Zahns herangezogen werden.

In einem ersten Schritt wird die relative Lage und Orientierung zwischen der Achse 1 des dentalen Implantats 2 und der optischen Achse 7 der Messkamera 6 bestimmt. Dazu wird ein mit der Messkamera 6 erfasstes Messbild durch geeignete Verfahren der Bildverarbeitung analysiert und die Lage und Orientierung der Messgeometrie 3a wird bestimmt. Von der Messgeometrie 3a kann dann auf eine Anschlussgeometrie 2a des Implantats 2 und damit auf die Lage und Orientierung des Implantats 2 selbst geschlossen werden.

In einem zweiten Schritt wird die klinische Situation im Bild analysiert. Dabei werden die Nachbarzähne 4, 5 berücksichtigt. Diese Analyse liefert eine Achse 8 relativ zur optischen Achse 7 der Messkamera 6. Ein Teil einer herzustellenden Implantatsuprastruktur 10, nämlich das Abutment 10, gleicht später den Winkel zwischen der Achse 1 des dentalen Implantats und der Achse 8 des Zahns aus. Die Implantatsuprastruktur besteht hier aus einem Abutment und einer nicht dargestellten Verblendung, ist also zweiteilig. Die Implantatsuprastruktur kann aber auch einteilig sein.

Der Ausgleichswinkel ergibt sich aus den gemessenen Winkeln zwischen der Achse 1 des dentalen Implantats und der optischen Achse 7 der Messkamera 6 und zwischen der optischen Achse 7 der Messkamera 6 und der Achse 8. Der Ausgleichswinkel ist also unabhängig von der optischen Achse 7 der Messkamera 6.

Sodann erfolgt die Konstruktion des Implantataufbaus im Computer, z.B. mit Hilfe eines CAD/CAM-Systems. Es entfällt somit die bisherige Notwendigkeit, ein aufwendiges Arbeitsmodell zu erstellen, anhand dessen der Zahnersatz hergestellt wurde.

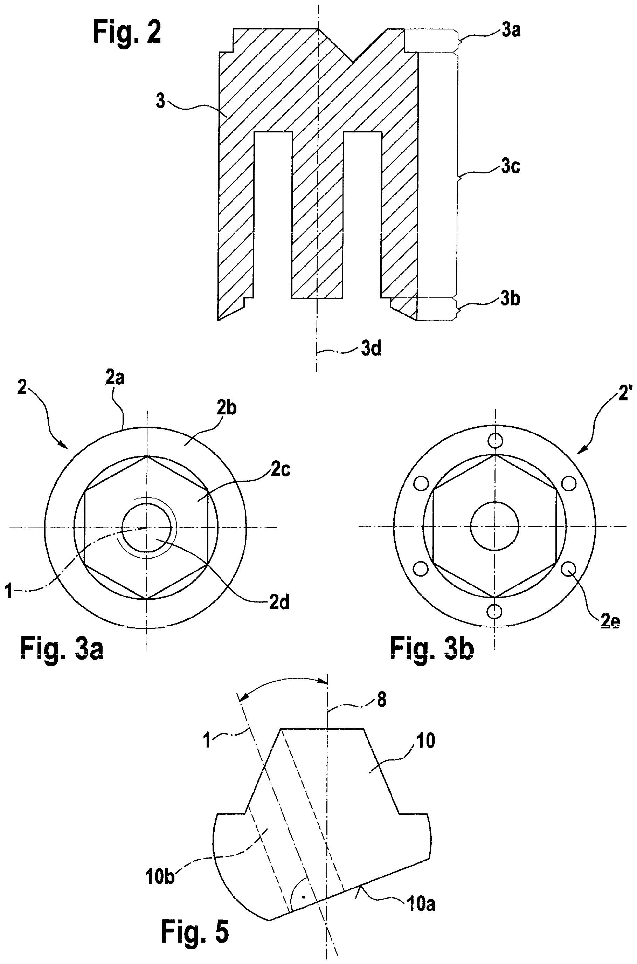

In Fig. 2 ist das Vermessungsteil 3 in Vergrößerung dargestellt. Das Vermessungsteil 3 weist an einem oberen Ende eine präzise gefertigte Messgeometrie 3a und an seinem unteren Ende eine Anschlussgeometrie 3b an das in Fig. 2 nicht dargestellte Implantat auf. Zwischen der Messgeometrie 3a und der Anschlussgeometrie 3b ist ein Distanzstück 3c vorhanden, sodass die Messgeometrie 3a vorteilhafterweise auf das Niveau der Okklusalfläche 9 aus Fig. 1 angehoben wird. Zwischen Anschlussgeometrie und Messgeometrie besteht ein eindeutiger geometrischer Zusammenhang. Dadurch muss nicht die Geometrie des Implantats direkt vermessen werden, sondern diese wird indirekt über die Messgeometrie vermessen.

Die Messgeometrie ist derart gestaltet, dass ihre Lage und Orientierung in einem aus den Messdaten gewonnenen Messbild durch geeignete Verfahren der Bildverarbeitung festgestellt werden kann.

Üblicherweise zeigen dentale Implantate keine eineindeutige, sondern eine mehrzählige Symmetrie, beispielsweise eine sechs- oder achtzählige. Die Vermessung der Messgeometrie lässt einen der Symmetrie entsprechenden eindeutigen Rückschluss auf Lage und Orientierung der Anschlussgeometrie zu.

Werden für unterschiedliche Anschlussgeometrien auch unterschiedliche Messgeometrien gewählt, können diese automatisch unterschieden werden. Wird für mehrere Anschlussgeometrien die gleiche Messgeometrie gewählt, muss in jedem Fall durch den Anwender eine Auswahl des verwendeten Implantats erfolgen.

Die Anschlussgeometrie 3b ist so geformt, dass sie formschlüssig auf das Implantat gesteckt werden kann.

In Fig. 3a ist eine Draufsicht auf das Implantat 2 gezeigt, sodass die Anschlussgeometrie 2a zum nicht dargestellten Vermessungsteil 3 hin zu erkennen ist. Es handelt sich hier um einen konischen Bereich 2b zur Zentrierung und um einen Bereich 2c mit Teilsymmetrien in Form eines Sechskants zur räumlichen Ausrichtung des anzuschließenden Bauteils. Die Befestigung erfolgt über ein Innengewinde 2d. Derartige Anschlussgeometrien sind Stand der Technik. Für unterschiedlich geformte Implantate, beispielsweise von unterschiedlichen Herstellern oder unterschiedlicher Bauart, sind unterschiedliche Anschlussgeometrien vorgesehen.

Wenn ein Implantat 2' neben der Anschlussgeometrie an die Suprastruktur noch über eine in Fig. 3b dargestellte Messgeometrie 2e verfügt, kann das Implantat mit einem geeigneten Messinstrument auch ohne Vermessungsteil direkt gemessen und die räumliche Ausrichtung bestimmt werden. Die Messgeometrie 2e weist halbkugelförmige Vertiefungen auf, deren Anzahl und Orientierung so bemessen ist, dass die räumliche Ausrichtung des Implantats bestimmt werden kann.

Darüber hinaus kann auch eine Kennung auf dem Implantat vorgesehen sein, die als Strichcode, Farbcode, Schrift oder in sonstiger Weise erfasst werden kann.

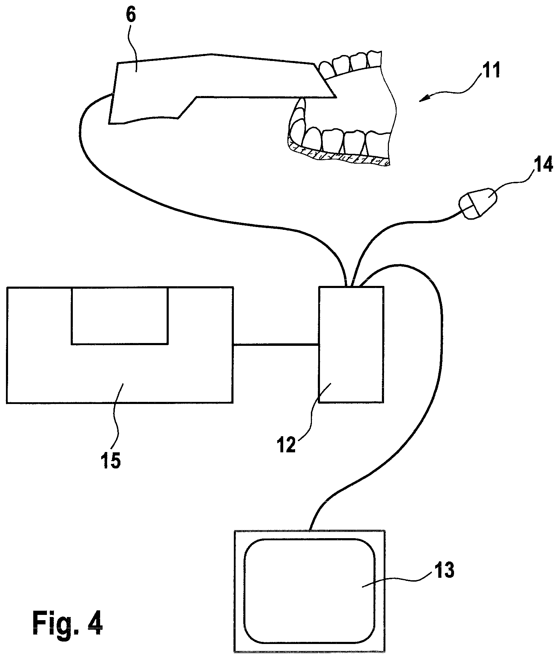

In Fig.4 ist eine Vorrichtung beschrieben, mit der das Verfahren gemäß der Erfindung ausgeführt werden kann. Die Vorrichtung umfasst eine Messkamera 6 zum Vermessen der klinischen Situation im Patientenmund 11. Die durch die Vermessung mit der Messkamera 6 gewonnen Messdaten werden in einer Auswerteeinheit 12 verarbeitet und gegebenenfalls gespeichert. Um die Messdaten für den Benutzer sichtbar zu machen, ist eine Anzeigeeinheit 15 vorgesehen, wobei die Auswerteeinheit 12 über Eingabemittel 14 gesteuert werden kann. Darüber hinaus ist eine Bearbeitungseinheit 13 vorgesehen, welche mit der Auswerteeinheit 12 zum Zweck des Datenaustausches verbunden ist. Selbstverständlich kann der Datenaustausch zwischen der Auswerteeinheit 12 und der Bearbeitungseinheit 13 auch auf andere Art und Weise erfolgen. In der Bearbeitungseinheit 13 lässt sich eine Implantatsuprastruktur 10 herstellen, welche die Winkellage des Implantats und die Einschubrichtung oder die gewünschte Okklusion berücksichtigt.

In Fig. 5 ist ein Ausführungsbeispiel für eine Implantatsuprastruktur in Form eines Abutments 10. Das Abutment 10 ist so geformt, dass ein Ausgleich zwischen der Implantatachse 1 und der Richtung der Achse 8 erfolgt. Zur Befestigung an der Anschlussgeometrie des Implantats ist ebenfalls eine Anschlussgeometrie 10a vorgesehen, wobei über eine Bohrung 10b eine Verschraubung mit dem Implantat möglich ist.

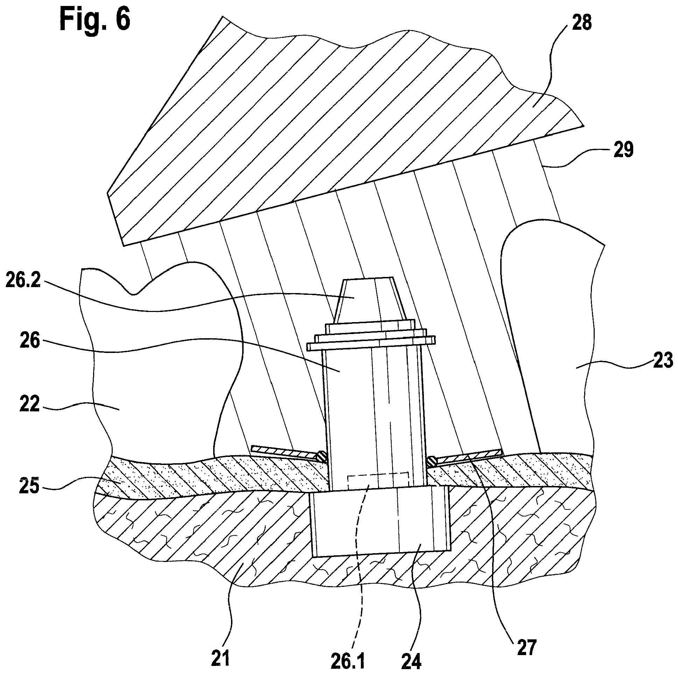

In Fig. 6 ist eine typische Präparationsstelle für eine Zahnrestauration dargestellt. In einen Kieferknochen 21 mit zu erhaltenden Zähnen 22, 23 ist zwischen den Zähnen 22, 23 ein Implantat 24 eingesetzt. Der Kieferknochen 21 wird überdeckt von Zahnfleisch 25, welches im Bereich des Implantats 24 von einem mit dem Implantat 24 über eine dazwischenliegende Passfläche als Bestandteil eines Positioniervorrichtung 6.1 verbundenen Aufsatzteil 26 durchbrochen ist.

Die Passfläche des Implantats ist regelmäßig so ausgebildet, dass ein guter Kraftschluss sowie eine relative Verdrehsicherheit gegeben sind und dass die notwendigen hygienischen Erfordernisse berücksichtigt sind. Historisch haben sich verschiedene Passflächen herausgebildet. Die Gegenstücke zu diesen Flächen werden nachgebildet und sind Bestandteil des Aufsatzteils 26.

Das Aufsatzteil 26 dient als Verschiebebahn für ein das Aufsatzteil umgebendes Ausgleichsteil 27, welches der Bestimmung und der Festlegung des Niveaus des Zahnfleischs (Gingiva-Niveau) dient. Es ist transversal an dem Aufsatzteil 26 entlang beweglich und kann nach dem Trennen des Aufsatzteils 26 vom Implantat 24 ausgewechselt werden. Das Ausgleichsteil kann als Gingiva-Teller bezeichnet werden und ist in seiner Orientierung flexibel einstellbar.

Das bewegliche Ausgleichsteil 27 befindet sich innerhalb des Schärfentiefebereichs einer intraoralen Messkamera 28, von welcher nur der vorderste Bereich mit der aus einem Messfenster austretenden Messstrahlung 29 dargestellt ist. Die Lage und die Orientierung des Ausgleichsteils 27 wird aus den gemessenen Daten gewonnen.

An dem Aufsatzteil 26 ist weiterhin ein Teilbereich 26.2 vorgesehen, der in seiner Struktur und/oder Codierung einem CAM-Gerät bekannt ist und der bei der Vermessung Rückschlüsse auf die Lage und Orientierung des Implantats zulässt. Unter CAM wird die Computer unterstützte Herstellung verstanden, die von dreidimensionalen Datensätzen ausgeht.

Durch das Ausgleichsteil 27, den sogenannten Gingivateller können auch in einem feuchten und blutenden Operationsgebiet die notwendigen Messdaten gewonnen werden.

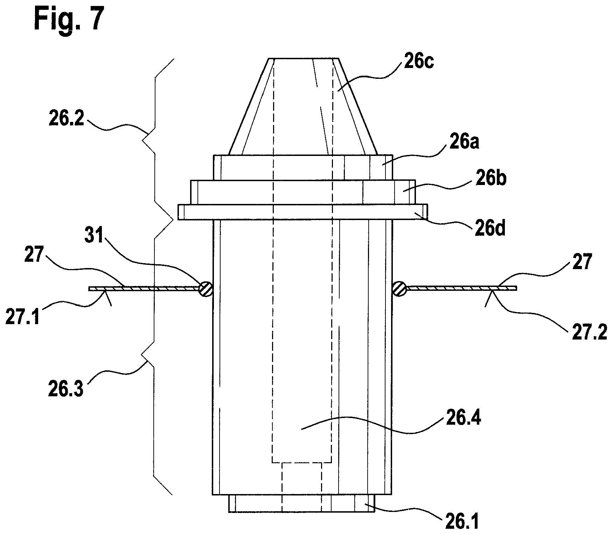

In Fig. 7 ist ein Aufsatzteil 26 in einer Seitenansicht dargestellt, wobei das Ausgleichsteil 27 ebenfalls gezeigt ist. An dem Aufsatzteil 26 lassen sich unterschiedliche Funktionsbereiche feststellen.

Am unteren Ende befindet sich die Positioniervorrichtung 26.1 zur lagegenauen Positionierung an einem nicht dargestellten Implantat. Die Passfläche des Implantats ist regelmäßig so ausgebildet, dass ein guter Kraftschluss sowie eine relative Verdrehsicherheit gegeben sind und dass die notwendigen hygienischen Erfordernisse berücksichtigt sind. Historisch haben sich verschiedene Passflächen herausgebildet. Die Gegenstücke zu diesen Flächen werden im Aufsatzteil nachgebildet. An diesem Ende erfolgt auch die Befestigung über eine angedeutete innenliegende Schraubverbindung 26.4.

Am oberen Ende ist der Teilbereich 26.2 mit codierten Informationen enthalten. Diese Informationen sind mittels unterschiedlicher Flächenbereiche codiert. Die Merkmale enthalten Informationen über die geometrische Orientierung der Passflächen des Implantats sowie über die Eigenschaften des eingesetzten Implantats (Hersteller, Größe, Typ etc.) enthalten. Diese können analysiert und ausgewertet werden.

Eine Fläche 26a, die konzentrisch zu der Rotationsachse des Implantats liegt, dient zur Bestimmung der Rotationsachse des Implantatkopfes.

Eine Fläche 26b ist in ihrem Durchmesser oder ihrem Verlauf auf das vorhandene Implantat angepasst und dient daher zur Bestimmung des Implantattyps.

Eine Fläche 26c dient zum automatischen Aufsuchen des Aufsatzteils 26 in dem durch Vermessung gewonnenen 3D-Datensatz und ist bei allen Aufsatzteilen identisch.

Über eine weitere Fläche 26d wird die Länge des nachstehend beschriebenen Distanzteils 26.3 codiert. Dadurch ist es ohne weiteres möglich, das Niveau des Implantatkopfes festzustellen, auch wenn der Implantatkopf selbst außerhalb des Messbereichs der Kamera liegt.

Die Flächen 26a-d sind als getrennte geometrische Bereiche an dem Aufsatzteil 26 vorgesehen sein. Das zugrunde liegende Prinzip besteht darin, dass es sich um Flächenelemente handelt, die dem CAD/CAM-Gerät in ihrer Struktur und/oder Kodierung bekannt sind und bei der Konstruktion von dentalen Passkörpern oder Suprastrukturen mittels Computer berücksichtigt werden.

Zwischen den beiden Enden des Aufsatzteils 26 liegt das Distanzteil 26.3, welches die Aufgabe hat, die zu vermessenden Teile 26a-d auf das Niveau der Okklusalfläche der Nachbarzähne zu bringen. Gleichzeitig dient es als Verschiebebahn für ein Ausgleichsteil 27, bezeichnet als Gingiva-Teller.

Dieser Gingiva-Teller 27 dient der Bestimmung und der Festlegung des Gingiva-Niveaus. Seitenflächen 27.1, 27.2 dienen der Auflage des Ausgleichsteils 27 auf dem gesunden Zahnfleisch, wie aus Fig. 6 ersichtlich ist. Das Ausgleichsteil 27 kann z.B. tellerartig gestaltet sein und die Anpassung an die Geometrie der Präparationsstelle wird erst durch die Verformung des Tellers vor Ort hergestellt. Alternativ lässt sich auch ein sattelförmiges Ausgleichsteil verwenden, das den Kieferquerschnitt seitlich stückweise umfasst.

Das Ausgleichsteil 27 ist transversal entlang des Distanzteils 26.3 beweglich, kann ausgewechselt werden und ist in seiner Orientierung flexibel einstellbar. Sind die dem zu versorgenden Implantat benachbarten Zähne weiter entfernt, kann das Ausgleichsteil 27 unter Berücksichtigung der anatomischen Gegebenheiten des Kieferbogens noch in Längsrichtung gekrümmt sein. Aufgrund der vergrößerten Darstellung lässt sich ebenfalls erkennen, dass eine das Aufsatzteil 26 umschließende Dichtung 31 angeordnet ist die verhindert, dass Blut und Speichel auf den Teilbereich 26.2 gelangt.

Zumindest der Teilbereich 26.2 ist vorzugsweise mit einem Material beschichtet, das einen hohen Rückstreukoeffizienten mit einer Lambert'schen Streuverteilung sowohl im sichtbaren wie im nahen Infrarot-Bereich des Spektrums aufweist. Auch das Ausgleichsteil 27 kann eine derartige Oberfläche aufweisen.

Im Zusammenhang mit der CAD/CAM gestützten Herstellung von Suprastrukturen erfolgt zunächst die Befestigung des mit dem Ausgleichteil 27 versehenen Aufsatzstücks 26 am Implantat 24 und anschließend die Verschiebung des Ausgleichsteils 27 auf das Niveau des Zahnfleischs 25. Anschließend erfolgt die Vermessung unter Herstellung eines dreidimensionalen Datensatzes.

Das Aufsatzteil umfasst einen Teilbereich 26.2 mit bekannter Geometrie. Diese Geometrie ermöglicht z. B. aufgrund ihrer Gleichmäßigkeit das automatisierte Auffinden solcher Strukturen in 3D-Datensätzen. Diese Geometrie ermöglicht weiter die Bestimmung der Orientierung des Implantats und der Lage nichtisotroper Teile des Implantats.

Diese Geometrie ermöglicht auch die Feststellung von Merkmalen wie z. B. Hersteller, Typ und Größe des verwendeten Implantats. Aufgrund der gewählten Länge des Distanzstücks 6e befindet sich dabei der Teilbereich innerhalb des Schärfentiefebereichs der Messkamera, und zwar auf dem Niveau der Okklusalfläche.

Durch Korrelation des 3D-Datensatzes mit einer digitalen Schablone, die einem Teil 6c des Aufsatzteils oder die ganze Vorrichtung widerspiegelt, kann das in dem gemessenen 3D-Datensatz enthaltene bzw. in dem daraus gewonnenen Bild sichtbare Aufsatzteil 26 automatisch aufgefunden werden. Ersatzweise kann auch eine Unterstützung durch den Nutzer z. B. mittels spezieller Eingaben vorgenommen werden.

Anschließend erfolgt die Bestimmung der Relativlage von Implantatkopf und Okklusalfläche der dem Implantat benachbarten Zähne. Anhand des z. B. tellerartigen Teils der Vorrichtung wird das Niveau der Gingiva bestimmt.

Für die Konstruktion der Suprastruktur wird die untere Bestimmungslinie eines Büchereizahns in mesio-distaler Richtung leicht unter das Niveau gelegt, das durch das Ausgleichteil 27 gegeben ist.

Dies kann sowohl unmittelbar nach Einbringung des Implantats in die Präparationsstelle als auch nach erfolgreicher Einheilung durchgeführt werden.

Vorteilhafterweise kann vor der Vermessung ein gegenüber dem Distanzstück bewegliches Ausgleichsteil zur Auflage auf dem die Zahnrestauration umgebenden Zahnfleisch positioniert und bei der Vermessung erfasst werden.

### Bezugszeichenliste

- 1: Achse des Implantats
- 2: Implantat
- 2a: Anschlussgeometrie des Implantats
- 2b,c,d: Bereich
- 3: Vermessungsteil
- 3a: Messgeometrie
- 3b: Anschlussgeometrie des Vermessungsteils
- 3c: Distanzstück
- 3d: Mittelachse
- 4: Nachbarzahn
- 5: Nachbarzahn
- 6: Messkamera
- 7: Achse des Vermessungssystems
- 8: Achse senkrecht zur Okklusalfläche
- 9: Okklusalfläche
- 10: Implantatsuprastruktur (Abutment)
- 10a: Anschlussgeometrie
- 10b: Bohrung
- 11: Patientenmund
- 12: Auswerteeinheit
- 13: Bearbeitungseinheit
- 14: Eingabemittel
- 15: Anzeigeeinheit
- 21: Kieferknochen
- 22, 23: Zähne
- 24: Implantat
- 25: Zahnfleisch
- 26: Aufsatzteil
- 27: Ausgleichsteil, Gingivateller
- 28: Messkamera
- 29: Messstrahlung
- 31: Dichtung

## Patentansprüche

1. Verfahren zur Bestimmung der Lage und Orientierung der Achse (1) eines direkt im Patientenmund befindlichen dentalen Implantats (2) für einen zu ersetzenden Zahn, wobei Messdaten, welche durch Vermessen der klinischen Situation des Implantats (2) und von Nachbarzähnen (4, 5) im Patientenmund gewonnen wurden, wobei an dem Implantat (2) eine Messgeometrie (3a) angebracht ist und wobei die Messgeometrie (3a) derart ausgebildet ist, dass sie einen Rückschluss auf die Lage und Orientierung einer Anschlussgeometrie (2a) des Implantats (2) zulässt, durch geeignete Verfahren analysiert werden und die Lage und Orientierung der Messgeometrie (3a) bestimmt wird, **dadurch gekennzeichnet, dass** anhand der Lagebestimmung der Messgeometrie (3a) die relative Lage und Orientierung zwischen der Achse (1) des dentalen Implantats und der Achse (7) der Vermessung bestimmt wird und anhand der Messgeometrie (3a) die Lage und die Orientierung des Implantats (2) selbst bestimmt wird und dass die klinische Situation des Implantats (2) und der Nachbarzähne (4, 5) in den Messdaten analysiert werden und dass eine Achse (8) unter Berücksichtigung der Nachbarzähne (4, 5) als Einschubachse oder unter Heranziehung von dem System aus einer Zahnbibliothek bekannten Beschaffenheiten als Zahnachse des zu ersetzenden Zahns relativ zur Achse (7) der Vermessung bestimmt wird.

2. Verfahren nach Anspruch 1, **dadurch gekennzeichnet, dass** die Messgeometrie (3a) an einem Vermessungsteil (3) mit einer Anschlussgeometrie für das Implantat (2) angebracht ist und dass aus der Messgeometrie die relative Lage und Orientierung der Anschlussgeometrie (2a) bestimmt wird.

3. Verfahren nach Anspruch 1 oder 2 , **dadurch gekennzeichnet, dass** ein Ausgleichswinkel einer Implantatsuprastruktur (10) zur Verbindung mit einem direkt im Patientenmund befindlichen dentalen Implantat (2) in seiner Lage und Orientierung bestimmt wird, der sich aus den gemessenen Winkeln zwischen der Achse (1) des dentalen Implantats (2) und der Achse (7) der Vermessung und zwischen der Achse (7) der Vermessung und der Achse (8) ergibt.

4. Verfahren nach einem der Ansprüche 1 bis 3, **dadurch gekennzeichnet, dass** die Messgeometrie (3a) und/oder die Anschlussgeometrie (2a) des Implantats mindestens einen Teilbereich aufweist, der so ausgebildet ist, dass sich aus der Analyse dieses Teilbereichs die Art des vorhandenen Implantats (2) ergibt.

5. Verfahren nach einem der Ansprüche 1 bis 4, **dadurch gekennzeichnet, dass** die Messgeometrie (3a) einen codierten Teilbereich aufweist und dass zwischen der Anschlussgeometrie (2a) und dem Teilbereich der Messgeometrie (3a) ein Distanzstück angebracht ist, das in seiner Länge so bemessen ist, dass der Teilbereich der Messgeometrie (3a) auf das Niveau der Okklusalfläche der Nachbarzähne gebracht ist, wobei im Teilbereich die Länge des Distanzstücks codiert ist.

6. Vorrichtung zur Durchführung des Verfahrens nach Anspruch 1, **dadurch gekennzeichnet, dass** in einer Auswerteeinheit (12) Messdaten, welche durch optisches Vermessen der klinischen Situation eines Implantats (2) und der Nachbarzähne im Patientenmund gewonnen wurden, wobei an dem Implantat (2) eine Messgeometrie (3a) angebracht ist und wobei die Messgeometrie (3a) derart ausgebildet ist, dass sie einen Rückschluss auf die Lage und Orientierung einer Anschlussgeometrie (2a) des Implantats (2) zulässt, durch geeignete Verfahren analysiert werden und die Lage und Orientierung der Messgeometrie (3a) bestimmt wird und in der Auswerteeinheit (12) anhand der Lagebestimmung der Messgeometrie (3a) die relative Lage und Orientierung der Achse (1) des dentalen Implantats (2) zu der Achse (7) der Vermessung bestimmt wird und in der Auswerteeinheit (12) ausgehend von der Messgeometrie (3a) die Lage und die Orientierung der Implantats (2) selbst bestimmt wird und in der Auswerteeinheit (12) die klinische Situation der Nachbarzähne (4, 5) in den Messdaten analysiert und eine Achse (8) unter Berücksichtigung der Nachbarzähne (4, 5) als Einschubachse oder unter Hinzuziehung von dem System aus einer Zahnbibliothek bekannten Beschaffenheiten als Zahnachse des zu ersetzenden Zahns relativ zur Achse (7) der Vermessung bestimmt wird.

7. Vorrichtung nach Anspruch 6, **dadurch gekennzeichnet, dass** in der Auswerteeinheit (12) ein Ausgleichswinkel bestimmt wird, der sich aus den gemessenen Winkeln zwischen der Achse (1) des dentalen Implantats (2) und der Achse (7) der Vermessung und zwischen der Achse (7) der Vermessung und der Achse (8) ergibt und eine Bearbeitungseinheit (13) vorgesehen ist, mit welcher die Implantatsuprastruktur (10) mit dem bestimmten Ausgleichswinkel herstellbar ist.

8. Vorrichtung nach Anspruch 6 oder 7, **dadurch gekennzeichnet, dass** die Messgeometrie (3a) und/oder die Anschlussgeometrie (2a) des Implantats mindestens einen Teilbereich aufweist, der so ausgebildet ist, dass sich aus der Analyse dieses Teilbereichs die Art des vorhandenen Implantats (2) ergibt.

9. Vorrichtung nach Anspruch 6 bis 8, **dadurch gekennzeichnet, dass** die Messgeometrie (3a) einen codierten Teilbereich aufweist und dass zwischen der Anschlussgeometrie (2a) und dem Teilbereich der Messgeometrie (3a) ein Distanzstück angebracht ist, das in seiner Länge so bemessen ist, dass der Teilbereich der Messgeometrie (3a) auf das Niveau der Okklusalfläche der Nachbarzähne gebracht ist, wobei im Teilbereich die Länge des Distanzstücks codiert ist.

## Claims

1. Method for determining the position and orientation of the axis (1) of a dental implant (2) located directly in the patient's mouth for a tooth to be replaced, wherein measurement data, obtained by measuring the clinical situation of the implant (2) and the adjacent teeth (4, 5) in the patient's mouth, wherein a measurement geometry (3a) is attached to the implant (2) and wherein the measurement geometry (3a) is configured such that it allows an inference to made about the position and orientation of a connecting geometry (2a) of the implant (2), is analyzed using suitable procedures and the position and orientation of the measurement geometry (3a) is determined, **characterized in that** the relative position and orientation between the axis (1) of the dental implant and the axis (7) of the measurement is determined using the position determination of the measurement geometry (3a) and the position and orientation of the implant (2) itself is determined with the aid of the measurement geometry (3a), and that the clinical situation of the implant (2) and the adjacent teeth (4, 5) in the measurement data is analyzed and that, taking into account the adjacent teeth (4, 5), an axis (8) is determined as an insertion axis or, using the system of qualities known from a tooth library, as the tooth axis of the tooth to be replaced relative to the axis (7) of the measurement.

2. Method according to Claim 1, **characterized in that** the measurement geometry (3a) is attached to a measurement part (3) having a connecting geometry for the implant (2) and that the relative position and orientation of the connecting geometry (2a) is determined from the measurement geometry.

3. Method according to Claim 1 or 2, **characterized in that** a compensation angle of an implant suprastructure (10) for connection to a dental implant (2) located directly in the patient's mouth is determined in terms of its position and orientation, which results from the measured angles between the axis (1) of the dental implant (2) and the axis (7) of the measurement and between the axis (7) of the measurement and the axis (8) .

4. Method according to any one of Claims 1 to 3, **characterized in that** the measurement geometry (3a) and/or the connecting geometry (2a) of the implant comprises at least one subregion, which is configured such that the type of the existing implant (2) emerges from the analysis of said subregion.

5. Method according to any one of Claims 1 to 4, **characterized in that** the measurement geometry (3a) comprises an encoded subregion and that a spacer is attached between the connecting geometry (2a) and the subregion of the measurement geometry (3a), the length of which is such that the subregion of the measurement geometry (3a) is brought to the level of the occlusal surface of the adjacent teeth, wherein the length of the spacer is encoded in the subregion.

6. Apparatus for carrying out the method according to Claim 1, **characterized in that** measurement data, obtained by optically measuring the clinical situation of an implant (2) and the adjacent teeth in the patient's mouth, wherein a measurement geometry (3a) is attached to the implant (2) and wherein the measurement geometry (3a) is configured such that it allows an inference to be made about the position and orientation of a connecting geometry (2a) of the implant (2), is analyzed in an evaluation unit (12) using suitable procedures and the position and orientation of the measurement geometry (3a) is determined, and, in the evaluation unit (12), the relative position and orientation of the axis (1) of the dental implant (2) to the axis (7) of the measurement is determined using the position determination of the measurement geometry (3a), and the position and orientation of the implant (2) itself is determined in the evaluation unit (12) on the basis of the measurement geometry (3a), and the clinical situation of the adjacent teeth (4, 5) in the measurement data is analyzed in the evaluation unit (12), and, taking into account the adjacent teeth (4, 5), an axis (8) is determined as an insertion axis or, using the system of qualities known from a tooth library, as the tooth axis of the tooth to be replaced relative to the axis (7) of the measurement.

7. Apparatus according to Claim 6, **characterized in that** a compensation angle, which results from the measured angles between the axis (1) of the dental implant (2) and the axis (7) of the measurement and between the axis (7) of the measurement and the axis (8), is determined in the evaluation unit (12), and a processing unit (13) is provided, with which the implant suprastructure (10) can be produced with the determined compensation angle.

8. Apparatus according to Claim 6 or 7, **characterized in that** the measurement geometry (3a) and/or the connecting geometry (2a) of the implant comprises at least one subregion, which is configured such that the type of the existing implant (2) emerges from the analysis of said subregion.

9. Apparatus according to Claim 6 to 8, **characterized in that** the measurement geometry (3a) comprises an encoded subregion and that a spacer is attached between the connecting geometry (2a) and the subregion of the measurement geometry (3a), the length of which is such that the subregion of the measurement geometry (3a) is brought to the level of the occlusal surface of the adjacent teeth, wherein the length of the spacer is encoded in the subregion.

## Revendications

1. Procédé pour la détermination de la position et de l'orientation de l'axe (1) d'un implant dentaire (2) se trouvant directement dans la bouche du patient pour une dent à remplacer, les données de mesure, qui ont été obtenues par une mesure de la situation clinique de l'implant (2) et des dents voisines (4, 5) dans la bouche du patient, une géométrie de mesure (3a) étant appliquée à l'implant (2) et la géométrie de mesure (3a) étant formée de telle façon qu'elle permet une conclusion sur l'emplacement et l'orientation d'une géométrie de raccordement (2a) de l'implant (2), étant analysées par un procédé approprié et la position et l'orientation de la géométrie de mesure (3a) étant déterminés, **caractérisé en ce qu'**au moyen de la détermination de position de la géométrie de mesure (3a) la position et l'orientation relatives entre l'axe (1) de l'implant dentaire et l'axe (7) de la mesure sont déterminées, et qu'au moyen de la géométrie de mesure (3a) la position et l'orientation de l'implant (2) même sont déterminées et que la situation clinique de l'implant (2) et des dents voisines (4, 5) sont analysées dans les données de mesure et qu'un axe (8) est déterminé, en tenant compte des dents voisines (4, 5), sous la forme d'un axe d'insertion ou, en utilisant des caractéristiques dentaires obtenues par le système à partir d'une bibliothèque dentaire, sous la forme d'un axe de dent de la dent à remplacer par rapport à l'axe de la mesure (7).

2. Procédé selon la revendication 1, **caractérisé en ce que** la géométrie de mesure (3a) est appliquée à un élément de mesure (3) comprenant une géométrie de raccordement pour l'implant (2), et que la position et l'orientation relatives de la géométrie de raccordement (2a) sont déterminées à partir de la géométrie de mesure.

3. Procédé selon la revendication 1 ou 2, **caractérisé en ce qu'**un angle de compensation d'une suprastructure implantaire (10), destinée à la connexion avec un implant (2) se trouvant directement dans la bouche du patient, est déterminé quant à sa position et orientation, lequel angle découle des angles mesurés entre l'axe (1) de l'implant dentaire (2) et l'axe (7) de la mesure et entre l'axe de la mesure (7) et l'axe (8).

4. Procédé selon l'une quelconque des revendications 1 à 3, **caractérisé en ce que** la géométrie de mesure (3a) et/ou la géométrie de raccordement (2a) de l'implant comprend au moins une zone partielle qui est formée de telle façon que le type de l'implant (2) existant découle de l'analyse de cette zone partielle.

5. Procédé selon l'une quelconque des revendications 1 à 4, **caractérisé en ce que** la géométrie de mesure (3a) comprend une zone partielle codée et qu'un élément d'espacement est disposé entre la géométrie de raccordement (2a) et la zone partielle de la géométrie de mesure (3a), dont la longueur est telle que la zone partielle de la géométrie de mesure (3a) est amenée au niveau de la surface occlusale des dents voisines, la longueur de l'élément d'espacement étant codée dans la zone partielle.

6. Dispositif pour l'exécution du procédé selon la revendication 1, **caractérisé en ce que**, dans une unité d'analyse (12), des données de mesure, lesquelles ont été obtenues par une mesure optique de la situation clinique d'un implant (2) et des dents voisines dans la bouche du patient, une géométrie de mesure (3a) étant appliquée à l'implant (2) et la géométrie de mesure (3a) étant formée de telle façon qu'elle permet une conclusion sur la position et l'orientation d'une géométrie de raccordement (2a) de l'implant (2), sont analysées par un procédé approprié et la position et l'orientation de la géométrie de mesure (3a) sont déterminées, la position et l'orientation relatives entre l'axe (1) de l'implant dentaire (2) et l'axe (7) de la mesure sont déterminées dans l'unité d'analyse (12) au moyen de la détermination de position de la géométrie de mesure (3a) et la position et l'orientation de l'implant dentaire (2) même sont déterminées à partir de la géométrie de mesure (3a) dans l'unité d'analyse (12) et la situation clinique des dents voisines (4, 5) dans les données de mesures est analysée dans l'unité d'analyse (12) et un axe (8) est déterminé, en tenant compte des dents voisines (4, 5), sous la forme d'un axe d'insertion ou, en utilisant des caractéristiques dentaires obtenues par le système à partir d'une bibliothèque dentaire, d'un axe de dent de la dent à remplacer par rapport à l'axe de la mesure (7).

7. Dispositif selon la revendication 6, **caractérisé en ce qu'**un angle de compensation est déterminé dans l'unité d'analyse (12), lequel découle des angles mesurés entre l'axe (1) de l'implant dentaire (2) et l'axe (7) de la mesure et entre l'axe (7) de la mesure et l'axe (8) et qu'une unité de traitement (13) est prévue, au moyen de laquelle la suprastructure implantaire (10) avec l'angle de compensation déterminé peut être produite.

8. Dispositif selon la revendication 6 ou 7, **caractérisé en ce que** la géométrie de mesure (3a) et/ou la géométrie de raccordement (2a) de l'implant comprend au moins une zone partielle qui est formée de telle façon que le type de l'implant (2) existant découle de l'analyse de cette zone partielle.

9. Dispositif selon l'une quelconque des revendications 6 à 8, **caractérisé en ce que** la géométrie de mesure (3a) comprend une zone partielle codée et qu'un élément d'espacement est disposé entre la géométrie de raccordement (2a) et la zone partielle de la géométrie de mesure (3a), dont la longueur est telle que la zone partielle de la géométrie de mesure (3a) est amenée au niveau de la surface occlusale des dents voisines, la longueur de l'élément d'espacement étant codée dans la zone partielle.
